# EUROPÄISCHE PATENTANMELDUNG

(11) **EP 4 505 969 A1**
(43) Veröffentlichungstag der Anmeldung: **12.02.2025**
(21) Anmeldenummer: 24188744.7
(22) Anmeldetag: 16.07.2024
(51) Int. Cl.: A61B 90/70, A61L 2/22, B05B 3/04

(54) **REINIGUNGSVORRICHTUNG**

(30) Priorität: 07.08.2023 DE 102023120943
(71) Anmelder: MMM Münchener Medizin Mechanik GmbH, 82152 Planegg (DE)
(72) Erfinder: FÜRG, Markus, 86684 Holzheim (DE); TSCHUI, Stefan, 86971 Peiting (DE); DEUTER, Martin, 82362 Weilheim (DE)
(74) Vertreter: Wittmann, Ernst-Ulrich

(57) **Zusammenfassung**

Eine Reinigungsvorrichtung wird bereitgestellt. Die Reinigungsvorrichtung umfasst eine Reinigungskammer und eine Sprüheinrichtung, die in der Reinigungskammer vorgesehen und eingerichtet ist, ein Reinigungsfluid auf die zu reinigenden Medizinprodukte zu sprühen. Die Sprüheinrichtung umfasst einen Schaft und einen Sprühkopf mit einem Düsenauslass zum Versprühen des Reinigungsfluids. Der Sprühkopf ist um eine Längsachse des Schafts herum drehbar an dem Schaft gestützt. Die Sprüheinrichtung ist derart eingerichtet, dass der Sprühkopf bei einem Versprühen des Reinigungsfluids dreht.

## Beschreibung

### Technisches Gebiet

Die vorliegende Erfindung betrifft eine Reinigungsvorrichtung zum Reinigen und Desinfizieren von wiederaufbereitbaren Medizinprodukten, insbesondere Endoskopen.

### Stand der Technik

Wiederaufbereitbare Medizinprodukte müssen nach dem Gebrauch, z.B. bei einer Untersuchung oder einer Operation, für eine Wiederverwendung aufbereitet werden. Ein wichtiger Schritt zur Aufbereitung der Medizinprodukte, wie zum Beispiel Endoskope, chirurgische Instrumente, usw. ist die Reinigung und Desinfektion. Dieser Aufbereitungsschritt muss nach geprüften, validierten Verfahren und in automatisch arbeitenden Reinigungsvorrichtungen erfolgen, die definierte Leistungskriterien erfüllen.

Bei den Reinigungsvorrichtungen handelt es sich um Reinigungs- und Desinfektionsgeräte, deren allgemeine Spezifikationen und Leistungsanforderungen in harmonisierten Normen festgelegt sind (z. B. Normenreihe DIN EN ISO 15883).

Eine wichtige Funktion der Reinigungsvorrichtungen ist die Abreinigung der Produktoberflächen von Rückständen, z.B. Blut. Zu diesem Zweck werden die Produkte üblicherweise in Siebschalen (Trays) gelegt und über einen längeren Zeitraum in einer Reinigungskammer der Reinigungsvorrichtung mit einem Reinigungsfluid besprüht. Der Ablauf des Reinigungsprozesses ist im Allgemeinen mehrstufig und erfolgt unter Verwendung von unterschiedlichen Reinigungsfluiden und Temperaturen. Verwendung findet hier beispielsweise Wasser, Weichwasser mit Reinigungschemie versetzt und demineralisiertes Wasser. Zur Reduzierung des Wasserverbrauchs wird das verwendete Wasser im Kreislauf für eine definierte Zeit umgewälzt und wieder verworfen. Am Ende des Reinigungsprozesses erfolgt ein letzter Schritt der Spülung mit demineralisiertem Wasser.

Herkömmlicherweise werden für die Außenreinigung der Medizinprodukte Sprüharme zur Verteilung des Reinigungsfluids über die zu reinigenden Medizinprodukte eingesetzt. Die Sprüharme sind in der Reinigungsvorrichtung oben und unten in der Reinigungskammer angeordnet und bei einer Beladung unter Verwendung von mehreren Siebschalen auf verschiedenen Ebenen zusätzlich zwischen den einzelnen Ebenen angeordnet. Die Sprüharme rotieren beim Versprühen des Reinigungsfluids innerhalb einer horizontalen Ebene, wodurch sichergestellt wird, dass das Reinigungsfluid an alle zu reinigende Stellen innerhalb der Reinigungskammer gelangt.

Die bekannten Reinigungsvorrichtungen haben jedoch den Nachteil, dass bei einer Beladung mit zu reinigenden Medizinprodukten eine Rotation der Sprüharme durch überstehende Teile oder überstehende Schläuche, die speziell bei der Reinigung von Endoskopen zur Innenreinigung der Medizinprodukte benötigt werden, blockiert werden kann. Bei einer Blockierung der Rotation der Sprüharme kann eine erforderliche Reinigungsleistung der Reinigungsvorrichtung nicht mehr sichergestellt werden.

Somit besteht ein Bedarf nach einer Reinigungsvorrichtung, bei der das Risko reduziert werden kann, dass eine Reinigungsleistung der Reinigungsvorrichtung durch überstehende Teile oder überstehende Schläuche der Beladung beeinflusst wird.

### Von der Erfindung zu lösende technische Aufgabe

Der vorliegenden Erfindung liegt daher die Aufgabe zugrunde, eine Reinigungsvorrichtung zum Reinigen und Desinfizieren von wiederaufbereitbaren Medizinprodukten, insbesondere Endoskopen, bereitzustellen, die eine verbesserte Zuverlässigkeit hinsichtlich einer Reinigungsleistung bietet.

### Offenbarung der Erfindung

Die Aufgabe wird erfindungsgemäß mit einer Reinigungsvorrichtung gelöst, die die Merkmale des unabhängigen Anspruchs 1 aufweist. Weitere vorteilhafte Ausgestaltungen sind in den abhängigen Ansprüchen definiert.

Die erfindungsgemäße Reinigungsvorrichtung umfasst eine Reinigungskammer und eine Sprüheinrichtung, die in der Reinigungskammer vorgesehen und eingerichtet ist, ein Reinigungsfluid auf die zu reinigenden Medizinprodukte zu sprühen. Die Sprüheinrichtung umfasst einen Schaft und einen Sprühkopf mit einem Düsenauslass zum Versprühen des Reinigungsfluids. Der Sprühkopf ist um eine Längsachse des Schafts herum drehbar an dem Schaft gestützt. Die Sprüheinrichtung ist derart eingerichtet, dass der Sprühkopf bei einem Versprühen des Reinigungsfluids dreht.

Durch die erfindungsgemäße Ausgestaltung der Sprüheinrichtung kann das Risiko, dass eine Drehung des Sprühkopfes während des Reinigungsprozesses durch überstehende Teile oder überstehende Schläuche in der Reinigungskammer beeinflusst wird, im Vergleich zur Verwendung herkömmlicher Sprüharme reduziert werden.

Des Weiteren kann durch den Einsatz der Sprüheinrichtung mit Sprühkopf der Verbrauch von Reinigungsfluid im Vergleich zur Verwendung herkömmlicher Sprüharme reduziert werden und dadurch ein Beitrag zur Ressourcenschonung geleistet werden.

Eine maximale Abmessung des Sprühkopfs in einer zu der Längsachse des Schafts rechtwinkligen Richtung kann nicht größer als das Vierfach, vorzugsweise das Dreifache, noch bevorzugter das Zweifache, einer maximalen Abmessung des Sprühkopfs entlang der Längsachse sein.

Durch die Reduzierte Abmessung des Sprühkopfs in einer zur Längsachse des Schafts rechtwinkligen Richtung, kann der Raum, der durch den sich drehenden Sprühkopf in der Reinigungskammer beansprucht wird, im Vergleich zur Verwendung herkömmlicher Sprüharme reduziert werden. Dadurch kann das Risiko, dass die Drehung des Sprühkopfs während des Reinigungsprozesses durch überstehende Teile oder überstehende Schläuche in der Reinigungskammer beeinflusst wird, reduziert werden.

Der Sprühkopf kann durch einen Rotationskörper ausgebildet sein.

Durch die eine Ausgestaltung des Sprühkopfes als Rotationskörper kann das Risiko, dass sich der Sprühkopf mit überstehenden Schläuchen in der Reinigungskammer verfängt und demzufolge eine Drehung des Sprühkopfs behindert wird, reduziert werden.

Eine Rotationsachse des als Rotationskörper ausgebildeten Sprühkopfs kann mit der Längsachse des Schafts übereinstimmen.

Des Weiteren kann der Sprühkopf derart durch den Rotationskörper ausgebildet und an dem Schaft drehbar gestützt sein, dass der drehende Sprühkopf nicht mehr Raum innerhalb der Reinigungskammer einnimmt als der ruhende Sprühkopf.

Dadurch kann der Platzbedarf der Sprüheinrichtung innerhalb der Reinigungskammer während des Reinigungsprozesses möglichst geringgehalten werden.

Der Sprühkopf kann kugelförmig oder zylinderförmig ausgebildet sein.

Der Düsenauslass kann durch eine in dem Sprühkopf vorgesehene Aussparung vorgesehen sein, die den Sprühkopf von innen nach außen durchdringt.

Durch die einfache geometrische Ausgestaltung des Sprühkopfes kann eine sehr gute Selbstreinigung der Sprüheinrichtung erreicht werden.

Die Sprüheinrichtung kann einen Sprühwinkel von 180° bis 360°, insbesondere 360°, aufweisen.

Durch den gewählten Sprühwinkelbereich kann die erforderliche Reinigungsleistung und eine erforderliche Selbstreinigung der Reinigungskammer gewährleistet werden.

Die Reinigungsvorrichtung kann eine Siebschale umfassen, die in der Reinigungskammer aufgenommen und eingerichtet ist, die zu reinigenden Medizinprodukte aufzunehmen.

Die zu reinigende Medizinprodukte können mit Hilfe der Siebschale optimal zur Reinigung in der Reinigungsvorrichtung gehalten und positioniert werden.

Die Reinigungsvorrichtung kann mehrere in der Reinigungskammer aufgenommene Siebschalen umfassen, die übereinander angeordnet sind.

Durch das Vorsehen von mehreren Siebschalen kann die Reinigungskapazität der Reinigungsvorrichtung erhöht werden und Energie gespart werden.

Die Reinigungskammer kann aus einer oberen Wand, einer der oberen Wand gegenüberliegenden unteren Wand, einer ersten Seitenwand und einer der ersten Seitenwand gegenüberliegenden zweiten Seitenwand gebildet sein. Die Sprüheinrichtung kann an der oberen Wand angebracht sein.

Durch die Anbringung der Sprüheinrichtung an der oberen Wand kann unter dem zusätzlichen Einfluss der Schwerkraft eine besonders gute und gleichmäßige Benetzung der zu reinigenden Medizinprodukte mit dem Reinigungsfluid erreicht werden.

Die Sprüheinrichtung kann an einer von der ersten und zweiten Seitenwand angebracht sein.

Durch die Anbringung der Sprüheinrichtung an einer Seitenwand kann eine bessere Benetzung der in der Reinigungskammer weiter unten angeordneten Teile der zu reinigenden Medizinprodukte erreicht werden.

Die Reinigungsvorrichtung kann mehrere in der Reinigungskammer vorgesehene Sprüheinrichtungen umfassen.

Durch die Bereitstellung mehrerer Sprüheinrichtungen kann eine Benetzung der zu reinigenden Medizinprodukte mit dem Reinigungsfluid erhöht und dadurch die Reinigungsleistung der Reinigungsvorrichtung, insbesondere bei höherer Beladung, verbessert werden.

Eine der mehreren Sprüheinrichtungen kann die an der oberen Wand angebrachte Sprüheinrichtung sein, eine weitere der mehreren Sprüheinrichtungen kann an der unteren Wand angebracht sein, und die übrigen Sprüheinrichtungen können an einer von der ersten und zweiten Seitenwand angebracht sein.

Durch das Anordnen der mehreren Sprüheinrichtungen an der oberen, unteren Wand und an einer der Seitenwände lassen sich Abschattungen durch Teile der zu reinigenden Medizinprodukte vermeiden. Eine Beeinflussung des Reinigungsergebnisses durch den Beladezustand der Reinigungsvorrichtung kann so verringert werden.

Die an der Seitenwand angebrachten Sprüheinrichtungen können jeweils an einer Position zwischen zwei unmittelbar übereinander angeordneten Siebschalen vorgesehen sein.

Durch die Anordnung der Sprüheinrichtungen zwischen zwei unmittelbar übereinander angeordneten Siebschalen erfolgt für jede Siebschale eine Benetzung mit dem Reinigungsfluid sowohl von oben als auch von unten, wodurch eine Benetzung der zu reinigenden Medizinprodukte verbessert werden kann. Durch die bessere Verteilung des Reinigungsfluids erfolgt eine vollständige Benetzung der Oberflächen schneller und unter Einsatz von weniger Reinigungsfluid und Reinigungszeit.

Die Sprüheinrichtung kann an einer Innenwand der Reinigungskammer abnehmbar angebracht sein.

Durch die abnehmbar angebrachte Sprüheinrichtung können die Montage- und Wartungsarbeiten der Reinigungsvorrichtung vereinfacht werden.

Der Schaft kann einen Fluideinlass und einen Fluidauslass aufweisen. Der Fluideinlass kann an einem Ende des Schafts vorgesehen sein, das an der Innenwand der Reinigungskammer abnehmbar angebracht ist, und der Fluidauslass kann an dem anderen Ende des Schafts vorgesehen sein. Der Sprühkopf kann an einem Abschnitt an dem anderen Ende des Schafts drehbar gestützt sein. Der Fluidauslass des Schafts kann mit dem Düsenauslass des Sprühkopfes in Verbindung stehen.

Der Düsenauslass kann bezüglich der Innenwand der Reinigungskammer in Richtung der Innenseite versetzt sein.

Durch den bezüglich der Innenwand nach innen versetzten Düsenauslass der Sprüheinrichtung kann ein Abstand zwischen der Innenwand der Reinigungskammer und dem Düsenauslass festgelegt werden. Somit kann eine Reinigung der Innenwand der Reinigungskammer auch in einem Bereich zuverlässig erfolgen, wo die Sprüheinrichtung an der Reinigungskammer angebracht ist.

Weitere Vorteile der vorliegenden Erfindung sind aus der nachfolgenden ausführlichen Beschreibung einer Ausführungsform und den angefügten Zeichnungen ersichtlich.

### Kurze Beschreibung der Zeichnungen

Figur 1 ist eine von einer Vorderseite aus gesehene schematische Schnittansicht einer Reinigungsvorrichtung gemäß einer Ausführungsform der vorliegenden Erfindung.
Figur 2 ist eine schematische Schnittansicht einer Sprüheinrichtung der in Figur 1 gezeigten Reinigungsvorrichtung.

### Beschreibung von mindestens einer beispielhaften Ausführungsform der Erfindung

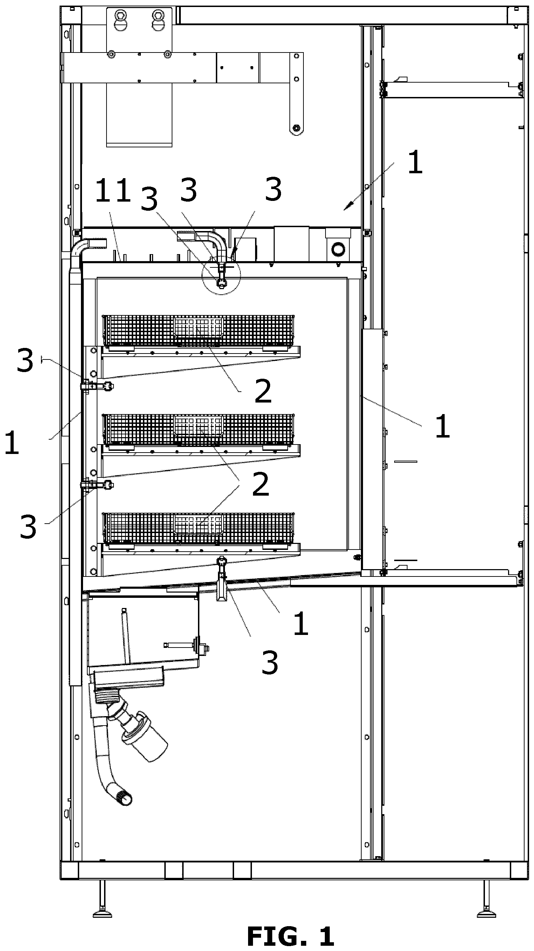

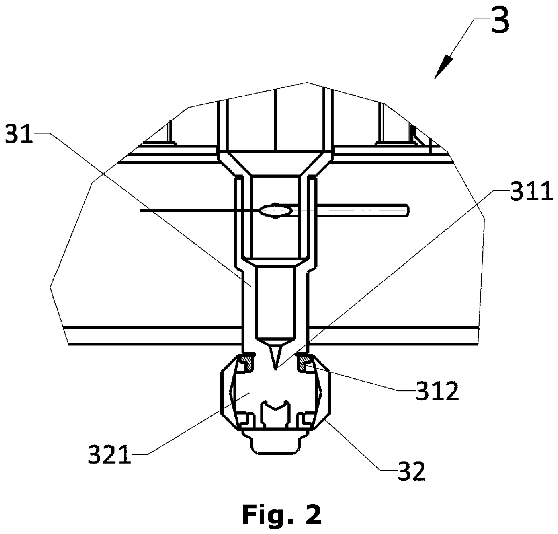

Nachfolgend wird unter Bezugnahme auf Figuren 1 und 2 eine beispielhafte Ausführungsform der vorliegenden Erfindung beschrieben.

Figur 1 zeigt eine schematische Schnittansicht der Reinigungsvorrichtung von einer Vorderseite aus gesehen. Die Reinigungsvorrichtung umfasst eine Reinigungskammer 1. In der vorliegenden Ausführungsform ist die Reinigungskammer 1 als im Wesentlichen rechteckige Kammer ausgebildet, die zwei entgegengesetzt angeordnete Öffnungen zum Be- bzw. Entladen der Reinigungskammer 1 aufweist. Eine erste Öffnung ist an der Vorderseite der in Figur 1 gezeigten Reinigungskammer 1 ausgebildet. Eine zweite Öffnung ist an der Rückseite der Reinigungskammer 1 ausgebildet.

Die Reinigungskammer 1 umfasst eine obere Wand 11, eine der oberen Wand 11 gegenüberliegende untere Wand 12, eine erste Seitenwand 13 und eine der ersten Seitenwand 13 gegenüberliegende zweite Seitenwand 14. Die Wände 11, 12, 13, 14 der Reinigungskammer 1 können als Einheit ausgebildet sein.

Die untere Wand 12 ist zu einem in der Reinigungskammer 1 vorgesehenen Ablauf hin leicht abfallend ausgebildet, um ein Ablaufen von Flüssigkeiten in der Reinigungskammer 1 zu unterstützen.

Die Reinigungskammer 1 umschließt einen im Wesentlichen quaderförmigen Kammerinnenraum. Die erste und die zweite Öffnung sind durch geeignet vorgesehene Türen (nicht gezeigt) verschließbar, sodass der Kammerinnenraum gegenüber der Umgebung hermetisch abgedichtet werden kann.

Die Angaben "oben", "unten", "Vorder...", "Rück..." und "Seiten..." beziehen sich auf Positionsangaben bei einer bestimmungsgemäßen Aufstellung der Reinigungsvorrichtung.

Die Reinigungsvorrichtung umfasst eine in der Reinigungskammer angeordnete Halteeinrichtung für mindestens eine Siebschale 2. Die Halteeinrichtung ist in der in Figur 1 gezeigten Ausführungsform derart gestaltet, dass mehrere horizontale Abstellflächen, die vertikal übereinander angeordnet sind, in der Reinigungskammer vorgesehen sind. Die Abstellflächen sind beispielsweise in der Gestalt von Gitterrosten ausgebildet, um sicherzustellen, dass Reinigungsfluid die Abstellflächen möglichst ungehindert durchdringen kann.

Mindestens eine Siebschale 2 kann in der Reinigungskammer 1 aufgenommenen werden. Die Siebschale 2 kann als einfacher Korb gestaltet sein, in den die zu reinigenden Medizinprodukte gelegt werden können. Die Siebschale 2 kann jedoch auch an spezielle zu reinigende Medizinprodukte, beispielsweise Endoskope, angepasst sein, sodass bestimmte Teile der zu reinigenden Medizinprodukte in der Siebschale 2 in einer Position gehalten werden können, die ein möglichst vollständiges Benetzen der Oberflächen der Medizinprodukte mit dem Reinigungsfluid während des Reinigungsvorgangs in der Reinigungsvorrichtung ermöglicht.

Die Siebschale 2 kann für eine Beladung mit den zu reinigenden Medizinprodukten aus der Reinigungskammer 1 genommen werden und nach dem Beladen wieder in die Reinigungskammer 1 eingeführt werden.

Die Reinigungskammer 1 ist des Weiteren so gestaltet, dass mehrere Siebschalen 2 in der Reinigungskammer 1 aufgenommen werden können. In der in Figur 1 gezeigten Ausführungsform können in der Reinigungskammer 1 drei Siebschalen 2 übereinander angeordnet werden, sodass eine oberste, mittlere und unterste Siebschale 2 in der Reinigungskammer 1 aufgenommen werden können. Insbesondere können die Siebschalen auf die durch die Halteeinrichtung ausgebildeten Abstellflächen gestellt werden.

Die Reinigungsvorrichtung umfasst des Weiteren eine Sprüheinrichtung 3, die in der Reinigungskammer 1 vorgesehen ist. Die Sprüheinrichtung 3 dient dazu, ein Reinigungsfluid innerhalb der Reinigungskammer 1 so zu verteilen, dass alle Außenflächen der zu reinigenden Gegenstände in der Reinigungskammer 1 sowie alle Wandinnenflächen der Reinigungskammer 1 möglichst gleichmäßig mit dem Reinigungsfluid benetzt werden.

Figur 2 zeigt eine schematische Schnittansicht der in der Reinigungskammer 1 angebrachten Sprüheinrichtung 3.

Die Sprüheinrichtung 3 hat einen im Wesentlichen rohrförmigen Schaft 31, der sich in Richtung einer Längsachse des Schafts 31 erstreckt. Die Richtung der Längsachse entspricht einer Axialrichtung des rohrförmigen Schafts 31. An einem Ende des Schafts 31 in der Axialrichtung ist ein Fluideinlass 311 und am anderen Ende des Schafts 31 ist ein Fluidauslass 312 vorgesehen. Das eine Ende des Schafts 31 mit dem Fluideinlass 311 ist an einen entsprechenden Anschluss in der Reinigungskammer 1 montiert, sodass der Fluideinlass 311 mit dem Anschluss der Reinigungskammer 1 in Verbindung steht. Der Anschluss ist an einer Innenwand der Reinigungskammer 1 (d.h. eine Wandfläche der Reinigungskammer 1, die dem von der Reinigungskammer 1 umschlossenen Kammerinnenraum zugewandt ist) vorgesehen und steht mit einer Reinigungsfluidzuführungseinrichtung der Reinigungsvorrichtung in Verbindung. Die Reinigungsfluidzuführungseinrichtung kann beispielsweise eine Fluidpumpe sein, die das Reinigungsfluid fördert. Der Fluidauslass 312 steht mit dem Fluideinlass 311 über einen in dem Schaft 31 ausgebildeten Fluidpfad in Verbindung.

Der Schaft 31 kann beispielsweise mittels einer Steckverbindung, die durch einen Splint gesichert wird, abnehmbar an dem Anschluss montiert werden. Dadurch können Montage-, Reinigungs- und Wartungsarbeiten der Reinigungsvorrichtung erleichtert werden.

Die Sprüheinrichtung 3 umfasst einen Sprühkopf 32. Der Sprühkopf 32 ist an dem anderen Ende des Schafts 31, an dem der Fluidauslass 312 vorgesehen ist, drehbar gestützt. Insbesondere ist der Sprühkopf 32 um die Längsachse des Schafts 31 herum drehbar gestützt. Dazu ist in dem Sprühkopf 32 ein Lagerloch ausgebildet, in dem ein Abschnitt an dem anderen Ende des Schafts 31 aufgenommen ist. Eine Lagerung kann beispielsweise mittels eines oder mehrerer Gleitlager erfolgen.

Der Sprühkopf 32 ist derart ausgebildet, dass die maximale Abmessung des Sprühkopfs 32 in einer zu der Längsachse des Schafts 31 rechtwinkligen Richtung nicht größer als das Zweifache der maximalen Abmessung des Sprühkopfs 32 entlang der Längsachse des Schafts 31 ist.

In der vorliegenden Ausführungsform ist der Sprühkopf 32 in der Gestalt eines Zylinders ausgebildet. Durch die Zylinderform ist der Sprühkopf 32 im Wesentlichen in der Gestalt eines Rotationskörpers ausgebildet. Das heißt, die Oberfläche (bzw. eine Außenfläche) des Rotationskörpers, der den Sprühkopf 32 ausbildet, wird durch Rotation einer erzeugenden Kurve um eine Rotationsachse gebildet, wobei die Rotationsachse in derselben Ebene wie die Kurve liegt.

Der Sprühkopf 32 kann beispielsweise auch durch andere Rotationskörper, z.B. eine Kugel, ausgebildet werden. Die vorliegende Erfindung ist jedoch nicht auf die Ausbildung des Sprühkopfs 32 als Rotationskörpers beschränkt.

Der Sprühkopf 32 ist an dem Schaft 31 drehbar gestützt, sodass die Rotationsachse des Rotationskörpers, der den Sprühkopf 32 ausbildet, mit der Längsachse des Schafts 31 übereinstimmt. Das heißt, eine Drehachse, um die sich der Sprühkopf 32 dreht, stimmt mit der Rotationsachse des Rotationskörpers überein, der den Sprühkopf 32 ausbildet. Folglich nimmt der sich drehende Sprühkopf 32 nicht mehr Raum ein als der ruhende Sprühkopf 32.

In dem Sprühkopf 32 ist mindestens ein Düsenauslass 321 vorgesehen. Der Düsenauslass 321 ist im Wesentlichen durch eine Aussparung ausgebildet, die den Sprühkopf 32 von einer radial innen gelegenen Seite in Richtung einer radial außen gelegenen Seite durchdringt. Der Düsenauslass 321 steht mit dem Fluidauslass 312 des Schafts 31 in Verbindung, sodass ein von der Reinigungsfluidzuführungseinrichtung gefördertes Reinigungsfluid über den Anschluss in der Reinigungskammer 1, den Fluideinlass 311 und den Fluidauslass 312 des Schafts 31 aus dem Düsenauslass 321 in die Reinigungskammer 1 gesprüht wird.

Der Sprühkopf 32 und der Düsenauslass 321 sind derart gestaltet, dass ein Durchströmen des Reinigungsfluids eine Drehung des Sprühkopfs 32 um die Drehachse herum bewirkt. Die Drehung bzw. die Drehgeschwindigkeit des Sprühkopfs 32 ist dabei von einer Strömungsgeschwindigkeit des Reinigungsfluids abhängig.

Insbesondere kann die Aussparung, die den Düsenauslass 321 ausbildet, bezüglich einer Radialrichtung des Sprühkopfs 32 geneigt sein. Durch die Neigung erfolgt ein Austritt des Reinigungsfluids aus dem Düsenauslass 321 schräg zu der Radialrichtung, wodurch das durchströmende Reinigungsfluid eine Kraft auf den Sprühkopf 32 ausübt, die den Sprühkopf 32 in Drehung versetzen kann. Alternativ kann eine Drehung des Sprühkopfs 32 auch durch andere Mittel, wie beispielsweise ein Turbinenrad, bewirkt werden.

Des Weiteren kann die Aussparung speziell gestaltet sein, um ein gewünschtes Sprühbild des Düsenauslasses 321 zu erreichen. Beispielsweise kann sich ein aus dem Düsenauslass 321 austretender Sprühstrahl kegelförmig aufweiten. Als Sprühstrahl kann beispielsweise ein Flachstrahl mit linienförmigen Sprühbild oder ein Vollkegelstrahl mit kreisförmigen Sprühbild eingesetzt werden.

Das Sprühbild des Sprühstrahls des Düsenauslasses 321 ergibt zusammen mit der Drehung des Sprühkopfs 32 einen Sprühwinkel der Sprüheinrichtung 3. Der Sprühwinkel beschreibt einen durch den Sprühstrahl erfassten Bereich einer Ebene, die die Drehachse des Sprühkopfs 32 umfasst, wobei die Drehachse die Winkelhalbierende des Sprühwinkels definiert.

Die Sprüheinrichtung 3 kann auch mehrere Düsenauslässe 321 aufweisen, die an dem Sprühkopf 32 verteilt angeordnet sind, um den gewünschten Sprühwinkel der Sprüheinrichtung 3 zu erreichen.

In der vorliegenden Ausführungsform umfasst der zylinderförmige Sprühkopf 32 zwei Düsenauslässe 321. Je ein Düsenauslass 321 ist an den beiden Axialenden des zylinderförmigen Sprühkopf 32 an abgeschrägten Randabschnitten an den äußeren Umfangsrändern vorgesehen. Des Weiteren sind die beiden Düsenauslässe 321 bezüglich einer Ebene, die rechtwinklig zu der Rotationsachse des Sprühkopfs 32 ist, symmetrisch angeordnet. Alternativ können die beiden Düsenauslässe 321 auch punktsymmetrisch bezüglich eines Axialmittelpunkts des Sprühkopfs 32 angeordnet sein.

Die in vorliegenden Ausführungsform verwendete Sprüheinrichtung 3 weist einen Sprühwinkel von 360° auf. Der Sprühwinkel kann jedoch auch im Bereich zwischen 180° bis 360° liegt.

Der Sprühkopf 32 mit dem mindestens einem Düsenauslass 321 ist durch den Schaft 31 mit Abstand von der Innenwand der Reinigungskammer 1 angeordnet. Der Abstand wird entlang der Längsachse des Schafts 31 gemessen und betrifft den geringsten Abstand des Sprühkopfs 32 zur Innenwand der Reinigungskammer 1. Dieser Abstand kann beispielsweise im Bereich von 20 mm bis 60 mm, bevorzugt von 30 mm bis 50 mm, liegen. Durch den Abstand des Sprühkopfs 32 zur Innenwand der Reinigungskammer 1 kann in Verbindung mit einem geeignet gewählten Sprühwinkel eine Selbstreinigung der Reinigungskammer 1, insbesondere der Innenwand der Reinigungskammer 1, erreicht werden.

In der vorliegenden Ausführungsform sind mehrere Sprüheinrichtungen 3 in der Reinigungskammer 1 angeordnet. Eine erste Sprüheinrichtung 3 ist an der oberen Wand 11 angeordnet. Die erste Sprüheinrichtung 3 ist im Wesentlichen an einer Position angeordnet, sodass sich die erste Sprüheinrichtung 3 mittig über der obersten Siebschale 2 befindet.

Eine zweite Sprüheinrichtung 3 ist an der unteren Wand 12 angeordnet. Die zweite Sprüheinrichtung 3 ist im Wesentlichen an einer Position angeordnet, sodass sich die zweite Sprüheinrichtung 3 mittig unter der untersten Siebschale 2 befindet.

Eine dritte Sprüheinrichtung 3 ist an einer der beiden Seitenwände 13, 14 (in der in den Figuren gezeigten Ausführungsform an der ersten Seitenwand 13) angeordnet. Die dritte Sprüheinrichtung 3 ist im Wesentlichen mittig zwischen der ersten und zweiten Öffnung angeordnet (d.h. eine Position der Sprüheinrichtung 3 bezüglich einer Horizontalrichtung bei bestimmungsgemäßer Aufstellung der Reinigungsvorrichtung). Die dritte Sprüheinrichtung 3 befindet sich auf einer Höhe, die im Wesentlichen einer Position zwischen einer Unterseite der obersten Siebschale 2 und einer Oberseite der mittleren Siebschale 2 entspricht (d.h. eine Position der Sprüheinrichtung 3 bezüglich einer Vertikalrichtung bei bestimmungsgemäßer Aufstellung der Reinigungsvorrichtung).

Eine vierte Sprüheinrichtung 3 ist an einer der beiden Seitenwände 13, 14 angeordnet. In der vorliegenden Ausführungsform sind die dritte und vierte Sprüheinrichtung 3 an derselben Seitenwand 13 angeordnet. Die Sprüheinrichtungen 3 können jedoch auch an verschiedenen Seitenwänden angeordnet sein.

Die vierte Sprüheinrichtung 3 ist im Wesentlichen mittig zwischen der ersten und zweiten Öffnung angeordnet. Die vierte Sprüheinrichtung 3 befindet sich auf einer Höhe, die im Wesentlichen einer Position zwischen einer Unterseite der mittleren Siebschale 2 und einer Oberseite der untersten Siebschale 2 entspricht.

Weder die Anzahl der Siebschalen 2, die in der Reinigungskammer 1 aufgenommen werden können, noch die Anzahl der in der Reinigungskammer 1 angeordneten Sprüheinrichtungen 3 sind beschränkt und können gemäß einer Größe der Reinigungskammer 1 und einer erforderlichen Reinigungsleistung angepasst werden.

Der Reinigungsfluidpfad der Reinigungsvorrichtung kann mit einem Filtersystem ausgestattet sein, das sicherstellt, dass keine Partikel die Drehung des Sprühkopfs 32 beeinträchtigen können. Für eine sichere Partikelzurückhaltung ist vorzugsweise ein Filtersystem zu wählen, das um Faktor zwei besser als für die jeweilige Sprüheinrichtung 3 erforderlich ist.

Eine Sprühleistung der Sprüheinrichtung 3 kann durch eine Kontrolle des von der Reinigungsfluidzuführungseinrichtung erzeugten Drucks überwacht werden.

Die obige Beschreibung ist nicht erschöpfend und die vorliegende Erfindung ist nicht auf die oben genannte(n) Ausführungsform(en) beschränkt. Der Fachmann wird erkennen, dass innerhalb des Umfangs der vorliegenden Erfindung verschiedene Abwandlungen und Kombinationen der in der/den obigen Ausführungsform(en) enthaltenen Merkmale möglich sind. Daher sollte der Umfang der vorliegenden Erfindung durch die beigefügten Ansprüche bestimmt werden.

## Patentansprüche

1. Reinigungsvorrichtung zum Reinigen und Desinfizieren von wiederaufbereitbaren Medizinprodukten, insbesondere Endoskopen, mit
einer Reinigungskammer (1), und
einer Sprüheinrichtung (3), die in der Reinigungskammer (1) vorgesehen und eingerichtet ist, ein Reinigungsfluid auf die zu reinigenden Medizinprodukte zu sprühen, wobei
die Sprüheinrichtung (3) einen Schaft (31) und einen Sprühkopf (32) mit einem Düsenauslass (321) zum Versprühen des Reinigungsfluids umfasst,
der Sprühkopf (32) um eine Längsachse des Schafts (31) herum drehbar an dem Schaft (31) gestützt ist, und
die Sprüheinrichtung (3) derart eingerichtet ist, dass der Sprühkopf (32) bei einem Versprühen des Reinigungsfluids dreht.

2. Reinigungsvorrichtung gemäß Anspruch 1, wobei
eine maximale Abmessung des Sprühkopfs (32) in einer zu der Längsachse des Schafts (31) rechtwinkligen Richtung nicht größer als das Vierfach, vorzugsweise das Dreifache, noch bevorzugter das Zweifache, einer maximalen Abmessung des Sprühkopfs (32) entlang der Längsachse ist.

3. Reinigungsvorrichtung gemäß Anspruch 1 oder 2, wobei
der Sprühkopf (32) durch einen Rotationskörper ausgebildet ist,
eine Rotationsachse des als Rotationskörper ausgebildeten Sprühkopfs (32) mit der Längsachse des Schafts (31) übereinstimmt, und
der Sprühkopf (32) derart durch den Rotationskörper ausgebildet und an dem Schaft (31) drehbar gestützt ist, dass der drehende Sprühkopf (32) nicht mehr Raum innerhalb der Reinigungskammer (1) einnimmt als der ruhende Sprühkopf (23).

4. Reinigungsvorrichtung gemäß einem der vorhergehenden Ansprüche, wobei der Sprühkopf (32) kugelförmig oder zylinderförmig ausgebildet ist.

5. Reinigungsvorrichtung gemäß einem der vorhergehenden Ansprüche, wobei
der Düsenauslass (321) durch eine in dem Sprühkopf (32) vorgesehene Aussparung vorgesehen ist, die den Sprühkopf (32) von innen nach außen durchdringt.

6. Reinigungsvorrichtung gemäß einem der vorhergehenden Ansprüche, wobei
die Sprüheinrichtung (3) einen Sprühwinkel von 180° bis 360°, insbesondere 360°, aufweist.

7. Reinigungsvorrichtung gemäß einem der vorhergehenden Ansprüche, wobei
die Reinigungsvorrichtung eine Siebschale (2) umfasst, die in der Reinigungskammer (1) aufgenommen und eingerichtet ist, die zu reinigenden Medizinprodukte aufzunehmen, oder
die Reinigungsvorrichtung mehrere in der Reinigungskammer (1) aufgenommene Siebschalen (2) umfasst, die übereinander angeordnet sind.

8. Reinigungsvorrichtung gemäß einem der vorhergehenden Ansprüche, wobei
die Reinigungskammer (1) aus einer oberen Wand (11), einer der oberen Wand (11) gegenüberliegenden unteren Wand (12), einer ersten Seitenwand (13) und einer der ersten Seitenwand (13) gegenüberliegenden zweiten Seitenwand (14) gebildet ist, und
die Sprüheinrichtung (3) an der oberen Wand (11) angebracht ist.

9. Reinigungsvorrichtung gemäß einem der Ansprüche 1 bis 7, wobei
die Reinigungskammer (1) aus einer oberen Wand (11), einer der oberen Wand (11) gegenüberliegenden unteren Wand (12), einer ersten Seitenwand (13) und einer der ersten Seitenwand (13) gegenüberliegenden zweiten Seitenwand (14) gebildet ist, und
die Sprüheinrichtung (3) an einer von der ersten und zweiten Seitenwand (13, 14) angebracht ist.

10. Reinigungsvorrichtung gemäß einem der vorhergehenden Ansprüche, wobei
die Reinigungsvorrichtung mehrere in der Reinigungskammer (1) vorgesehene Sprüheinrichtungen (3) umfasst.

11. Reinigungsvorrichtung gemäß Anspruch 8 und 10, wobei
eine der mehreren Sprüheinrichtungen (3) die an der oberen Wand (11) angebrachte Sprüheinrichtung (3) ist, eine weitere der mehreren Sprüheinrichtungen (3) an der unteren Wand (12) angebracht ist, und die übrigen Sprüheinrichtungen an einer von der ersten und zweiten Seitenwand (13, 14) angebracht sind.

12. Reinigungsvorrichtung gemäß Anspruch 7 und 9 oder 7 und 11, wobei
die an der Seitenwand (13, 14) angebrachten Sprüheinrichtungen (3) jeweils an einer Position zwischen zwei unmittelbar übereinander angeordneten Siebschalen (2) vorgesehen sind.

13. Reinigungsvorrichtung gemäß einem der vorhergehenden Ansprüche, wobei
die Sprüheinrichtung (3) an einer Innenwand der Reinigungskammer (1) abnehmbar angebracht ist.

14. Reinigungsvorrichtung gemäß einem der vorhergehenden Ansprüche, wobei
der Schaft (31) einen Fluideinlass (311) und einen Fluidauslass (312) aufweist,
der Fluideinlass (311) an einem Ende des Schafts (31) vorgesehen ist, das an der Innenwand der Reinigungskammer (1) abnehmbar angebracht ist,
der Fluidauslass (312) an dem anderen Ende des Schafts (31) vorgesehen ist,
der Sprühkopf (32) an einem Abschnitt an dem anderen Ende des Schafts (31) drehbar gestützt ist, und
der Fluidauslass (312) mit dem Düsenauslass (321) in Verbindung steht.

15. Reinigungsvorrichtung gemäß einem der vorhergehenden Ansprüche, wobei der Düsenauslass (321) bezüglich der Innenwand der Reinigungskammer (1) in Richtung der Innenseite versetzt ist.
